Europäisches Patentamt

European Patent Office

Office européen des brevets

(11) Publication number: **0 078 138**
**B2**

(12) # NEW EUROPEAN PATENT SPECIFICATION

(45) Date of publication of the new patent specification: 26.09.90

(51) Int. Cl.[5]: **A 61 K 7/08**

(21) Application number: **82305517.3**

(22) Date of filing: **18.10.82**

(54) **Shampoo composition.**

(30) Priority: **24.10.81 GB 8132141**

(43) Date of publication of application:
**04.05.83 Bulletin 83/18**

(45) Publication of the grant of the patent:
**22.10.86 Bulletin 86/43**

(45) Mention of the opposition decision:
**26.09.90 Bulletin 90/39**

(84) Designated Contracting States:
**AT BE CH DE FR GB IT LI LU NL SE**

(73) Proprietor: **BEECHAM GROUP PLC
Beecham House Great West Road
Brentford Middlesex TW8 9BD (GB)**

(72) Inventor: **Straw, Philip Leigh
62 Marlborough Avenue
Ruislip Middlesex (GB)**
Inventor: **Wilkins, Anne Lilian
34 Harrowdene Gardens
Teddington Middlesex (GB)**

(74) Representative: **Russell, Brian John et al
Beecham Pharmaceuticals Great Burgh Yew
Tree Bottom Road
Epsom Surrey KT18 5XQ (GB)**

(56) References cited:
EP-A-0 004 821
AU-B- 437 869
DE-A-2 053 109          GB-A-1 359 492
FR-A-2 113 128          GB-A-1 435 624
GB-A- 813 514           US-A-3 580 853
GB-A- 891 027           US-A-3 785 985
GB-A-1 202 716          US-A-3 962 418
                        US-A-3 980 767

The file contains technical information
submitted after the application was filed and
not included in this specification

(56) References cited:
MODERN PACKAGING, vol. 34, no. 6, February
1961, pages 98-100, New York, US, "Stripe
toothpaste"

CHEMICAL ABSTRACTS, vol. 96, no. 16, April
1982, page 447, no. 129586g, Columbus, Ohio,
US

"Manufacturing Chemist", Feb. 1984, p. 13
"Performance Characteristics and Solution
Properties of Surfactants in Shampoos", Int.
Journal of Cosmetic Science 1, 1979, pp. 73-75
Int. Journal of Cosmetic Science 1 (1979)
pp.73-75

EP 0 078 138 B2

## Description

The present invention relates to a shampoo composition, and particularly to a shampoo composition containing an antidandruff agent.

Antidandruff agents, such as zinc pyridine thione (zinc omadine; zinc 2-pyridine thiol-N-oxide, hereinafter referred to as ZPT) are frequently included in shampoo compositions for combatting dandruff. Many materials suitable for this type of use tend to be insoluble in aqueous solution, and hence shampoo compositions containing such agents have an opaque or cloudy appearance. Transparent or translucent shampoo compositions are visually attactive but are rendered opaque by the addition of these agents.

By means of the present invention, it is possible to combine the visual attractiveness of a transparent or translucent shampoo composition with the advantages of an antidandruff agent.

According to the present invention, there is provided a shampoo composition containing from 5 to 25% w/w of anionic detergent, 0.5 to 7% of a thickening agent, which thickening agent has secondary detergent properties, an antidandruff agent, and an agent for improving the cosmetic condition of the hair after washing, comprising a first transparent or translucent detergent-containing gel body and a second substantially opaque detergent-containing gel body which is contiguous with and co-extrudable with the first gel body, whereby when the composition is discharged from a container the two gel bodies remain in contact with each other and form a self-supporting gel structure, and wherein the second gel body contains the antidandruff agent, and one or both gel bodies contain(s) the said agent for improving the cosmetic condition of the hair after washing. The composition is suitably contained in a tube or other container which is preferably collapsible.

The consistencies of the two gel bodies should be sufficiently firm to hold the bodies together in position, and yet be easily extrudable through, for example, the nozzle of a tube. Preferably, the two gel bodies have substantially identical rheological properties.

The preferred antidandruff agent is ZPT, and this is suitably present in the composition in an amount of 0.1 to 2.5% by weight of the total composition. The weight ratios of the two bodies can vary within wide limits. For example, the weight ratio of the first gel body to the second gel body may be from about 1:9 to 9:1. A particularly suitable ratio is 1:1. The distribution of the two gel bodies can also vary widely in order to give different visual patterns. For example, one gel body may be completely contained within the other gel body, or one gel may be partly contained in the other gel body to form a stripe or stripes.

The desired rheological properties of the two gel bodies may be achieved by incorporating suitable thickening materials into the bodies.

It has been found desirable to use as a thickening agent a material which has secondary detergent and/or foam stabilising properties. One or more such foam stabiliser/thickener materials may be present in each gel body, and preferred materials are lauric isopropanolamide or coconut monoethanolamide. The amount of such a foam stabiliser/thickener is from 0.5 to 7.0% by weight of the total composition.

Preferably each gel body contains one or more agents designed to improve the cosmetic condition of the hair after shampooing, by enhancing feel and gloss and reducing electrostatic flyaway. Such agents may comprise wholly or in part the detergent materials described above as providing a foam stabilising or thickening effect, or may comprise other agents with surface active properties, or certain polymeric materials. Suitable surface active materials are certain salts of ethoxylated fatty alcohol phosphate esters, particularly an oleyl alcohol derived material marketed under the trade name Briphos O3D. Suitable polymeric materials are cationic cellulosic resins marketed under the trade name Polymer JR 125,400 or 30M or quaternary of a vinyl pyrrolidone copolymer marketed under the trade name Gafquat 755N. A particularly effective material is a cationic guar gum sold under the trade name Jaguar C-13-S by Meyhall Chemicals Ltd. This may be present in either or both gel bodies in an amount from 0.1 to 5.0% by weight of the total composition.

The compositions according to the invention may include any conventional anionic detergent well known in the art, for example alkali metal, ammonium or hydroxyalkylamine salts of alkyl sulphates or alkyl ether sulphates, alkyl benzene sulphonates, alkyl sulphones, α-alkenyl sulphonates, polyoxyethylenealkyl sulphonates and polyoxyethylenealkylphenylsulphates. A preferred detergent comprises sodium lauryl ether sulphate.

The compositions of the invention may also contain various conventional accessory ingredients such as perfumes, colouring materials and preservatives.

Both gel bodies may be coloured, if desired, provided the first gel body remains transparent or translucent.

The compositions of the invention may be made by charging a suitable container with two gel bodies, using the method and apparatus described in British Patent Specification No. 962,757.

The present invention will now be illustrated by the following Examples.

# EP 0 078 138 B2

Example 1

| Ingredients | %w/w | |
|---|---|---|
| | First gel body | Second gel body |
| SLES (28%) | 70.0 | 67.20 |
| Lauric isopropanolamide | 3.0 | 2.88 |
| Coconut monoethanolamide | 3.0 | 2.88 |
| Cationic guar gum | 0.5 | 0.48 |
| Zinc pyrithione | — | 2.0 |
| Perfume, dyes, preservatives, etc | q.s. | q.s. |
| Water to | 100.0% | 100.0% |

SLES (28%) refers to a 28% w/v aqueous solution of sodium lauryl ether sulphate.
The weight ratio of the two gel bodies is 1:1.

Example 2

| Ingredients | %w/w | |
|---|---|---|
| | First gel body | Second gel body |
| SLES (28%) | 65.0 | 65.0 |
| Lauric isopropanolamide | 2.88 | 2.88 |
| Coconut monoethanolamide | 2.88 | 2.88 |
| Cationic guar gum | 0.3 | 0.30 |
| Zinc pyrithione | — | 4.0 |
| Perfume, dyes, preservatives, etc | q.s. | q.s. |
| Water to | 100.0% | 100.0% |

The weight ratio of the first gel body to the second gel body is 3:1.

3

Example 3

| Ingredients | %w/w | |
|---|---|---|
| | First gel body | Second gel body |
| SLES (28%) | 75.0 | 73.0 |
| Lauric isopropanolamide | 2.75 | 2.68 |
| Coconut monoethanolamide | 2.75 | 2.68 |
| Cationic guar gum | 0.7 | 0.62 |
| Zinc pyrithione | — | 1.33 |
| Perfume, dyes, preservatives, etc | q.s. | q.s. |
| Water to | 100.0% | 100.0% |

The weight ratio of the first gel body to the second gel body is 1:3.

Example 4

| Ingredients | %w/w | |
|---|---|---|
| | First gel body | Second gel body |
| SLES (28%) | 70.0 | 67.2 |
| Ethoxylated fatty alcohol | 3.0 | 2.88 |
| Sodium salt of an ethoxylated oleyl phosphate ester | 3.0 | 2.88 |
| Zinc pyrithione | — | 2.0 |
| Perfume, dyes, preservatives, etc | q.s. | q.s. |
| Water to | 100.0% | 100.0% |

The weight ratio of the first gel body to the second gel body is 1:1.

**Claims**

1. A shampoo composition containing from 5 to 25% w/w of anionic detergent, 0.5 to 7% of a thickening agent, which thickening agent has secondary detergent properties, an antidandruff agent, and an agent for improving the cosmetic condition of the hair after washing, comprising a first transparent or translucent detergent-containing gel body and a second substantially opaque detergent-containing gel body which is contiguous with and co-extrudable with the first gel body, whereby when the composition is discharged from a container the two gel bodies remain in contact with each other and form a self-supporting gel structure, and wherein the second gel body contains the antidandruff agent, and one or both gel bodies contain(s) the said agent for improving the cosmetic condition of the hair after washing.

2. A composition according to claim 1, in which the two gel bodies have substantially identical rheological properties.

3. A composition according to claim 1 or claim 2, in which the anti-dandruff agent comprises zinc pyridine thione.

4. A composition according to any one of claims 1 to 3, in which the weight ratio of the first gel body to the second gel body is from 1:9 to 9:1.

5. A composition according to any one of claims 1 to 4, in which one gel body is partly contained within the other gel body to form a stripe or stripes.

6, A composition according to any one of claims 1 to 5, in which one or both gel bodies contain a cosmetic condition improving agent selected from salts of ethoxylated fatty alcohol phosphate esters, cationic cellulosic resins, quaternary vinyl pyrrolidone copolymers, and cationic guar gums.

7. A container, containing a composition according to any one of claims 1 to 6, which includes a nozzle through which the composition may be extruded.

8. A container according to claim 7, in the form of a collapsible tube.

**Patentansprüche**

1. Eine Haarwaschmittelzusammensetzung, enthaltend von 5 bis 25% Gew./Gew. eines anionischen Detergens, 0,5 bis 7% eines Verdickungsmittels, welches Verdickungsmittel, das zusätzlich Eigenschaften eines Detergens hat, ein Mittel gegen Schuppen und ein Mittel zur Verbesserung des kosmetischen Zustandes des Haars nach dem Waschen, umfassend einen ersten durchsichtigen oder durchscheinenden, detergenshaltigen Gelkörper und einen zweiten, im wesentlichen undurchsichtigen, detergenshaltigen Gelkörper, der angrenzend an und zusammen mit dem ersten Gelkörper extrudierbar ist, wobei, wenn die Zusammensetzung aus einem Behälter entnommen wird, die beiden Gelkörper in Berührung miteinander bleiben und eine selbsttragende Gelstruktur bilden, und in welcher der zweite Gelkörper ein Mittel gegen Schuppen enthält, und ein Gelkörper oder beide Gelkörper besagtes Mittel zur Verbesserung des kosmetischen Zustandes des Haars nach dem Waschen enthält bzw. enthalten.

2. Eine Zusammensetzung nach Anspruch 1, in welcher die beiden Gelkörper im wesentlichen identische rheologische Eigenschaften haben.

3. Eine Zusammensetzung nach Anspruch 1 oder Anspruch 2, in welcher das Mittel gegen Schuppen Zink-pyridin-thion umfaßt.

4. Eine Zusammensetzung nach irgendeinem der Ansprüche 1 bis 3, in welcher das Gewichtsverhältnis des ersten Gelkörpers zum zweiten Gelkörper 1:9 bis 9:1 ist.

5. Eine Zusammensetzung nach irgendeinem der Ansprüche 1 bis 4, in welcher ein Gelkörper teilweise in dem anderen Gelkörper unter Bildung eines Streifens oder von Streifen enthalten ist.

6. Eine Zusammensetzung nach irgendeinem der Ansprüche 1 bis 5, in welcher ein Gelkörper oder beide Gelkörper ein den kosmetischen Zustand verbesserndes Mittel enthält, das aus Salzen äthoxylierter Fettalkohol-phosphatester, kationischer cellulosehaltiger Harze, quaternärer Vinylpyrrolidon-Copolymer und kationischer Guargummen ausgewählt ist.

7. Ein Behälter, enthaltend eine Zusammenstzung nach irgendeinem der Ansprüche 1 bis 6, der eine Düse einschließt, durch die die Zusammensetzung extrudiert werden kann.

8. Ein Behälter nach Anspruch 7 in Form einer zusammenfaltbaren Tube.

**Revendications**

1. Composition pour shampooings contenant de 5 à 25% en p/p d'un détergent anionique, 0,5 à 7% d'un agent épaississant, lequel agent épaississant à des propriétés détergentes secondaires, un agent antipelliculaire et un agent pour améliorer l'état cosmétique des cheveux après lavage, comprenant une première masse de gel transparente ou translucide contenant un détergent et une seconde masse de gel sensiblement opaque contenant un détergent, qui est contiguë à la première masse de gel et peut être extrudée conjointement avec elle, de telle sorte que lorsque la composition est évacuée d'un récipient, les deux masses de gel demeurent en contact l'une avec l'autre et forment une structure de gel autoportante et dans laquelle la seconde masse de gel contient un agent anti pelliculaire et l'une ou les deux masse de gels contiennent cet agent pour améliorer l'état cosmétique des cheveux après le lavage.

2. Composition suivant la revendication 1, dans laquelle les deux masses de gels ont des propriétés rhéologiques sensiblement identiques.

3. Composition suivant la revendication 1 ou la revendication 2, dans laquelle l'agent antipelliculaire comprend de la pyridinethione de zinc.

4. Composition suivant l'une quelconque des revendications 1 à 3, dans laquelle le rapport en poids entre la première masse de gel et la seconde masse de gel va de 1:9 à 9:1.

5. Composition suivant l'une quelconque des revendications 1 à 4, dans laquelle une masse de gel est contenue partiellement à l'intérieur de l'autre masse de gel pour former un ou plusieurs filets ou bandes.

6. Composition suivant l'une quelconque des revendications 1 à 5, dans laquelle l'une ou les deux masses de gels contiennent un agent améliorant l'état cosmétique choisi parmi des sels de phosphates-esters d'alcools gras éthoxylés, des résines cellulosiques cationiques, des dérivés quaternaires de copolymères de vinylpyrrolidone et des gommes guar cationiques.

7. Récipient contenant une composition suivant l'une quelconque des revendications 1 à 6, qui comporte une buse ou un orifice par lequel la composition peut être extrudée.

8. Récipient suivant la revendication 7, sous la forme d'un tube écrasable.